# EUROPEAN PATENT APPLICATION

(11) **EP 2 022 481 A1**
(43) Date of publication of application: **11.02.2009**
(21) Application number: 07015481.0
(22) Date of filing: 07.08.2007
(51) Int. Cl.: A61K 8/73, A61K 8/898, A61Q 5/02, A61Q 5/12

(54) **Conditioning composition for keratinic fibres**

(71) Applicant: KPSS-Kao Professional Salon Services GmbH, 64297 Darmstadt (DE)
(72) Inventor: Hermes, Frank, 64665 Alsbach-Hähnlein (DE); Molenda, Michael, 60487 Frankfurt (DE); Förster, Sabine, 64319 Pfungstadt (DE)

(57) **Abstract**

The present invention relates to a conditioning composition for keratin fibres especially human hair comprising particular cationic cellulose polymer and a cationic or cationizable silicone compound.

## Description

The present invention relates to a conditioning composition for keratin fibres especially human hair comprising particular cationic cellulose polymer and a cationic or cationizable silicone compound.

Conditioning composition for keratin fibres have been known for a long time. They are usually applied after cleansing hair and rinsed off from hair, although leave-in conditioning compositions have recently become available and gains more and more popularity among consumers under so called "easy to use" concept.

The function of a hair conditioner as understood by the consumer is first off all detangle hair and furthermore give hair smoothness, elasticity, volume and body, and shine. In spite of availability of various conditioning compositions, there are still needs for improvements. The need for improvement is especially high for consumers either having fine hair, meaning hair with a smaller diameter, or damaged hair for which a high level of conditioning is needed for smoothing and therewith easing further handling for example combability. On the other hand, when a conditioning composition with high level of conditioning ingredients is used, hair looses its volume and body and often lacks shine and elasticity.

Present invention starts from the above mentioned problems and provides a conditioning composition which effectively detangles hair without loosing volume and body, and with enhanced smoothness and shine.

The present inventor has surprisingly found out that an aqueous hair conditioning composition comprising at least one cationic cellulose polymer and a cationic or cationizable silicone compound improves hair combability, smoothness, shine, elasticity and more interestingly hair treated with such a composition has enough volume and body and excellently manageable.

Accordingly, the first subject of the present invention is an aqueous composition for conditioning keratin fibres especially human hair comprising at least one cationic cellulose polymer and at least one cationic or cationizable silicone compound.

Second subject matter of the present invention is the use of the composition comprising at least one cationic cellulose and at least one cationic or cationizable silicone compound for conditioning hair.

The third subject matter of the present invention is use of the composition for improving smoothness, shine, elasticity volume and body of hair.

The forth subject matter of the present invention is use of the composition for improving smoothness, shine, elasticity volume and body of damaged hair. With the term damaged hair it is meant that hair is undergone chemical treatments such as oxidative colouring, bleaching and/or permanent shaping or damaged with the effects of the environmental influences.

The fifth subject matter of the present invention is use of the composition for improving smoothness, shine, elasticity volume and body of fine hair.

Compositions of the present invention comprise at least one cationic cellulose polymer. The cationic cellulose polymers are well known with the CTFA names, for example, Polyquaternium-4, Polyquaternium-10, Polyquaternium-2a, Polyquaternium-67 and Polyquaternium-72 and commercially available from various suppliers under various trade names.

Preferred cationic cellulose derivatives are Polyquaternium-4, Polyquaternium-10, Polyquaternium-24, Polyquaternium-67 and Polyquaternium-72 and more preferred ones are Polyquaternium-4, Polyquaternium-24, Polyquaternium-67 and Polyquaternium-72. The most preferred is Polyquaternium-67.

Cationic cellulose are comprised in the compositions of the present invention at a concentration of 0.01 - 2.5%, preferably 0.05 - 2%, more preferably 0.1 - 1.5 and most preferably 0.25 -1% by weight calculated to total composition.

Compositions of the present invention comprise at least cationic or cationizable silicone compound. With the term cationizable silicone compound it is meant that free amino groups either primary, secondary or tertitary can be protonated and present a cationic group.

Cationizable silicone compounds are known with the CTFA name amodimethicone and available from Dow Corning under the trade names, for example, DC 949 emulsion, and DC Q2-8220. Suitable ones are also those derivatives of amodimethicone such as bis (C13-15 alkoxy) PG-amodimethicone.

Further cationizable silicone compound suitable for the compositions of the present invention is polysilicone-9 (CTFA adopted name) available under the trade name Elastomer OS from Kao Corporation. In this respect reference is made to the cationic polymers disclosed in EP-A 524 612 and EP-A 640 643. They are quatemized products of graft polymers from organopolysiloxanes and polyethyl oxazolines also suitable for the compositions of the present invention.

Cationic silicone compound which is known with the CTFA adopted name Quaternium-80 and is available under the trade name Abil Quat from Degussa.

Most preferred is the compound bis (C13-15 alkoxy) PG-amodimethicone.

Cationizable or cationic silicones are included into the compositions of the present invention at a concentration of 0.01 to 5%, preferably 0.05 to 3%, and more preferably 0.1 to 2.5 and most preferably 0.25 to 2% by weight calculated to total composition.

The compositions of the present invention can be either a conditioning -cleansing composition - shampoo - or a conditioning composition typically used after use of cleansing compositions.

Oily substances are selected from such as silicone oils, either volatile or non-volatile, natural and synthetic oils. Among silicone oils those can be added to the compositions include dimethicone, dimethiconol, polydimethylsiloxane, DC fluid ranges from Dow Corning, natural oils such as olive oil, almond oil, avocado oil, wheatgerm oil, ricinus oil and the synthetic oils, such as mineral oil, isopropyl myristate, palmitate, stearate and isostearate, oleyl oleate, isocetyl stearate, hexyl laurate, dibutyl adipate, dioctyl adipate, myristyl myristate and oleyl erucate.

Non-ionic conditioning agents may be polyols such as glycerin, glycol and derivatives, polyethyleneglycoles known with trade names Carbowax PEG from Union Carbide and Polyox WSR range from Amerchol, polyglycerin, polyethyleneglycol mono or di fatty acid esters having general formula

R₁ CO (O CH₂ CH₂)ₙ OH

or

R₁ CO (O CH₂ CH₂)ₙ O OC R₂

where R₁ and R₂ are independent from each other saturated, unsaturated or branched or non-branched alkyl chain with 7 to 21 C atoms and n is typically 2 - 100.

In one of the preferred from of the present invention, conditioning compositions comprise at least one cationic polymer as conditioning agent. Suitable cationic polymers are those of best known with their CTFA category name Polyquaternium Typical examples of those Polyquaternium 6, Polyquaternium 7, Polyquaternium 11, Polyquaternium 16, Polyquaternium 22, Polyquaternium 28, and Polyquaternium-70.

As well those polymers known with their CTFA category name Quaternium are suitable. Those are for example Quaternium-8, Quaternium-14, Quaternium-15 Quaternium-18, Quaternium-22, Quaternium-24, Quaternium-26, Quaternium-27, Quaternium-30, Quaternium-33, Quaternium-53, Quaterntum-60, Quaternium-61, Quaternium-72, Quaternium-78, Quaternium-81, Quaternium-82, Quaternium-83 and Quaternium-84.

It has further been found out that cationic guar gum known with trade name Jaguar from Rhône-Poulenc and chemically for example Guar hydroxypropyl trimonium chloride, are also additional suitable cationicpolymer. Furthermore, chitosan and chitin can also be included in the compositions as cationic natural polymers. In this context reference is also made to the cationic polymers disclosed in DE 25 21 960, 28 11 010, 30 44 738 and 32 17 059, as well as to the products described in EP-A 337 354 on pages 3 to 7. It is also possible to use mixtures of various cationic polymer.

The cationic polymers also include the quaternized products of graft polymers from organopolysiloxanes and polyethyl oxazolines described in EP-A 524 612 and EP-A 640 643.

Conditioning compositions of the present invention can comprise additionally one or more cationic surfactant(s) as conditioner presented with the general formula 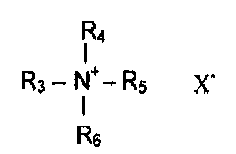 where R₃ is a saturated or unsaturated, branched or non-branched alkyl chain with 8-22 C atoms or

R₇ CO NH (CH₂)ₙ

where R₇ is saturated or unsaturated, branched or non-branched alkyl chain with 7-21 C atoms and n has value of 1 - 4,
or

R₈ CO O (CH₂)ₙ

where R₈ is saturated or unsaturated, branched or non-branched alkyl chain with 7-21 C atoms and n has value of 1 - 4, and
R₄ is hydrogen or unsaturated or saturated, branched or non-branched alkyl chain with 1 - 4 C atoms or

R₇ CO NH (CH₂)ₙ

or

R₈ CO O (CH₂)ₙ

where R₇, R₈ and n are same as above.

R₅and R₆ are hydrogen or lower alkyl chain with 1 to 4 carbon atoms, and X is anion such as chloride, bromide, methosulfate.

Typical examples of those ingredients are cetyltrimethyl ammonium chloride, steartrimonium chloride, behentrimonium chloride, stearamidopropyl trimonuim chloride, dioleoylethyl dimethyl ammonium methosulfate, dioleoylethyl hydroxyethylmonium methosulfate.

Amido amines may as well be used as a conditioning cationic surfactant in the compositions of the present invention. Typical non-limiting example is stearamidopropylamine known with a trade name Tego Amid S18 from Degussa. The compositions according to the invention may also comprise further conditioning substances such as protein hydrolyzates and polypeptides, e.g., keratin hydrolyzates, collagen hydrolyzates of the type "Nutrilan^{R}" or elastin hydrolyzates, as well as also in particular plant protein hydrolyzates, optionally, cationized protein hydrolyzates, e.g., "Gluadin^{R}".

Typical concentration range for any of those conditioners of cationic polymers, silicon oil and derivatives and cationic surfactants can be 0.01 - 10% by weight, preferably 0.01 - 7.5% by weight, more preferably 0.05 - 5% and most preferably 0.1 - 3% by weight calculated to the total composition. It should be noted that especially non-cleansing conditioning type of the products contain higher concentrations of the above mentioned concentrations of the cationic surfactants which at the same time if desired can be emulsifying agent. In cleansing and conditioning type of preparations, concentration of cationic surfactants is lower.

Conditioning composition can comprises organic solvents such as ethanol, propanol, isopropanol, benzyl alcohol, benzyloxyethanol, ethoxydiglycol, alkylene carbonates such as ethylene carbonate and propylene carbonate, phenoxyethanol, butanol, isobutanol, cyclohexane, cyclohexanol, hexyleneglycol, ethylenecarbonate, propyleneglycol, poypropyleneglycols, ethyleneglycol monoethylether, ethylene glycol monobutyl ether, ethylene glycol monophenyl ether, 1-phenylethylalcohol, 2-phenylethylalcohol, o-methoxyphenol. The most preferred ones are benzyloxyethenol and polypropylene glycols. Concentration of organic solvents should not exceed 10% by weight, preferably in the range of 0.1 to 7.5%, more preferably 0.1 to 5% by weight and most preferably 0.1 to 3% by weight calculated to total composition.

Conditioning compositions of the present invention can be a cleansing composition (cleansing-conditioning composition). Cleansing conditioning compositions of the present invention comprise at least one surfactant selected from anionic, non-ionic and/or amphoteric or zwitterionic surfactants at a concentration range of 5 to 50%, preferably 5 to 40% and more preferably 5 to 30%, and most preferably 5 to 25% by weight, calculated to the total composition.

In an embodiment of the present invention cleansing conditioning composition of the present invention, comprises at least one anionic, at least one nonionic surfactant. More preferably the compositions further comprise additionally at least one amphoteric surfactant.

Anionic surfactants suitable within the scope of the invention are preferably present in an amount from 1 to about 30%, preferably 2 to 20% and most preferably 2 - 15%, by weight, calculated to the total composition.

These are anionic surfactants of the sulfate, sulfonate, carboxylate and alkyl phosphate type, especially, of course, those customarily used in shampoo compositions, for example, the known C₁₀-C₁₈-alkyl sulfates, and in particular the respective ether sulfates, for example, C₁₂-C₁₄-alkyl ether sulfate, lauryl ether sulfate, especially with 1 to 4 ethylene oxide groups in the molecule, monoglyceride (ether) sulfates, fatty acid amide sulfates obtained by ethoxylation and subsequent sulfatation of fatty acid alkanolamides, and the alkali salts thereof, as well as the salts of long-chain mono- and dialkyl phosphates constituting mild, skin-compatible detergents.

Additional anionic surfactants useful within the scope of the invention are α-olefin sulfonates or the salts thereof, and in particular alkali salts of sulfosuccinic acid semiesters, for example, the disodium salt of monooctyl sulfosuccinate and alkali salts of long-chain monoalkyl ethoxysulfosuccinates.

Suitable surfactants of the carboxylate type are alkyl polyether carboxylic acids and the salts thereof of the formula

R₉ - (C₂H₄O)ₙ - O - CH₂COOX,

wherein R₉ is a C₈-C₂₀-alkyl group, preferably a C₁₂-C₁₄-alkyl group, n is a number from 1 to 20, preferably 2 to 17, and X is H or preferably a cation of the group sodium, potassium, magnesium and ammonium, which can optionally be hydroxyalkyl-substituted, as well as alkyl amido polyether carboxylic acids of the general formula 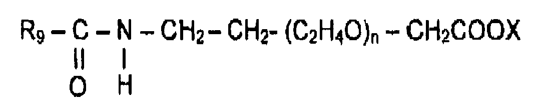 wherein R₉ and X have the above meanings, and n is in particular a number from 1 to 10, preferably 2.5 to 5.

Such products have been known for some time and are on the market, for example, under the trade name "AKYPO^{®}" and "AKYPO-SOFT^{®}".

Also useful are C₈-C₂₀-acyl isethionates, alone or in admixture with other anionic surfactants, as well as sulfofatty acids and the esters thereof.
It is also possible to use mixtures of several anionic surfactants, for example an ether sulfate and a polyether carboxylic acid or alkyl amidoether carboxylic acid.

An overview of the anionic surfactants used in liquid body cleansing compositions can furthermore be found in the monography of K. Schrader and A. Domsch, "Cosmetology - Theory and Practice", 2005, Verlag für chemische Industrie, Augsburg - Germany, pp. II-8 -II-19.

Further suitable anionic surfactants are also C₈-C₂₂-acyl aminocarboxylic acids or the water-soluble salts thereof. Especially preferred is N-lauroyl glutamate, in particular as sodium salt, as well as, for example, N-lauroyl sarcosinate, N-C₁₂-C₁₈-acyl asparaginic acid, N-myristoyl sarcosinate, N-oleoyl sarcosinate, N-lauroyl methylalanine, N-lauroyl lysine and N-lauroyl aminopropyl glycine, preferably in form of the water-soluble alkali or ammonium, in particular the sodium salts thereof, preferably in admixture with the above-named anionic surfactants.

Further surfactants in the shampoo compositions according to the invention are nonionic surfactants in admixture with anionic surfactants.

These are described in Schrader, I.c., on pages 600-601 and pp. 694-695. Especially suited are alkyl polyglucosides of the general formula

R₁₀-O(R₁₁O)ₙ-Zₓ,

wherein R₁₀ is an alkyl group with 8 to 18 carbon atoms, R₁₁ is an ethylene or propylene group, Z is a saccharide group with 5 to 6 carbon atoms, n is a number from 0 to 10 and x is a number between 1 and 5.

These alkyl polyglucosides have recently become known in particular as excellent skin-compatible, foam improving agents in liquid detergents and body cleansing compositions, and are present in an amount from about 1 % to 15 %, in particular from 1 % to 10 % by weight, calculated to the total composition.

Mixtures of anionic surfactants and alkyl polyglucosides as well as the use thereof in liquid body cleansing compositions are already known, for example, from EP-A 70 074. The alkyl polyglucosides disclosed therein are basically also suited within the scope of the present invention; as well as the mixtures of sulfosuccinates and alkyl polyglucosides disclosed in EP-A 358 216.

Further nonionic surfactant components are, for example, long-chain fatty acid mono-and dialkanolamides, such as coco fatty acid monoethanolamide and myristic fatty acid monoethanolamide, which can also be used as foam enhancers, preferably in amounts from about 1 % to about 5 % by weight.

Further additionally useful nonionic surfactants are, for example, the various sorbitan esters, such as polyethylene glycol sorbitan stearic acid ester, fatty acid polyglycol esters or poly-condensates of ethyleneoxide and propyleneoxide, as they are on the market, for example, under the trade name "Pluronics^{R}", as well as fatty alcohol, ethoxylates.

Further suitable nonionic surfactants are amineoxides which may be present in an amount from 0.25 % to 5 % by weight, calculated to the total composition.

Such amineoxides are state of the art, for example C₁₂-C₁₈- alkyl dimethyl amineoxides such as lauryl dimethyl amineoxide, C₁₂-C₁₈- alkyl amidopropyl or -ethyl amineoxides, C₁₂-C₁₈ -alkyl di(hydroxyethyl) or (hydroxypropyl) amineoxides, or also amineoxides with ethyleneoxide and/or propyleneoxide groups in the alkyl chain. Such amineoxides are on the market, for example, under the trade names "Ammonyx^{®}", "Aromox^{®}" or "Genaminox^{®}".

Further nonionic surfactants useful in the compositions according to invention are C₁₀-C₂₂-fatty alcohol ethoxylates at a concentration of 0.5 to 10%, preferably 0.5 to 5% by weight, calculated to total composition. Especially suited are C₁₀-C₂₂-fatty alcohol ethers, the alkyl polyglycol ethers known by the generic terms "Laureth", "Myristeth", "Oleth", "Ceteth", "Deceth", "Steareth" and "Ceteareth" according to the CTFA nomenclature, including addition of the number of ethylene oxide molecules, e.g., "Laureth-16":

The average degree of ethoxylation thereby ranges between about 2.5 and about 25, preferably about 10 and about 20.

As further surfactant component, the compositions according to the invention can also contain amphoteric or zwitterionic surfactants, for example in an amount from about 0.5 % to about 15 %, preferably from about 1 % to about 10 %, by weight, calculated to the total composition. It has especially been found out that addition of zwitterionic or amphoteric surfactants enhances foam feeling in terms of creaminess, foam volume and as well as skin compatibility is improved. For achieving milder formulations anionic surfactant, especially of sulphate types, to amphoteric surfactant ratio should be in the range of 10:1 1 to 1:1, preferably 5:1 1 to 1:1.

Useful as such are in particular the various known betaines such as alkyl betaines fatty acid amidoalkyl betaines and sulfobetaines, for example, lauryl hydroxysulfobetaine; long-chain alkyl amino acids, such as cocoaminoacetate, cocoaminopropionate and sodium cocoamphopropionate and acetate have also proven suitable.

In detail, it is possible to use betaines of the structure 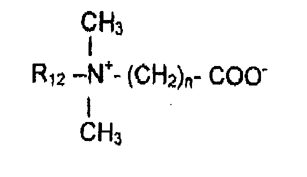 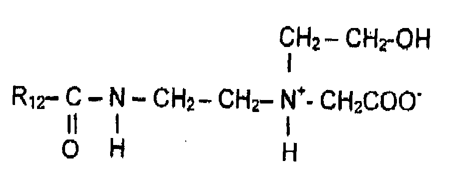 wherein R₁₂ is a C₈-C₁₈-alkyl group and n is 1 to 3;
sulfobetaines of the structure 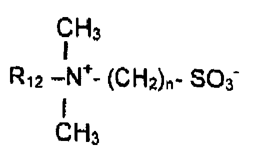 wherein R₁₂ and n are same as above;
and amidoalkyl betaines of the structure 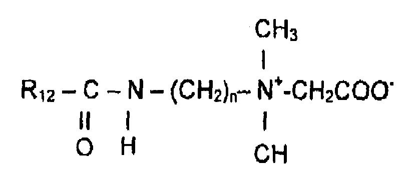 wherein R₁₂ and n are same as above.

Solubilizers may be added to the compositions, in particular cleansing compositions especially when oily substances are chosen as conditioning agents and fragrance oils with highly lipophilic properties. Typical solubilizers may be hydrogenated castor oil known with the trade mark Cremophor RH series from BASF. It should be noted that as well the surfactant mixture can be a good solubilizer for fragrance oils. Typical concentration of the solubilizers can be in the range of 0.01 - 2% by weight, preferably 0.1 - 1% by weight, calculated to total composition.

Compositions of the present invention especially those applied after cleansing hair can be in the form of thickened gels and emulsion. In case that composition is a thickened gel it comprises additionally at least one thickening agent. Such thickening agents are for example, cellulose derivatives such as hydroxyethyl or hydroxyethyl cellulose, further cationic polymers such as polyquaternium-37, and anionic polymers such as acrylate copolymers as long as no compatibility problem raises.

Preferably the composition of the present invention used after cleansing hair is aqueous oil in water emulsion.

Compositions of the present invention comprise at least one fatty alcohol with an alkyl chain length of 92 to 24 C atoms, preferably 14 to 22 C atoms and more preferably 16 to 22 C atoms. Non-limiting examples are lauryl alcohol, myristyl alcohol, cetyl alcohol stearyl alcohol and behenyl alcohol. Composition can also comprise more than one fatty alcohol such as any mixture of the above mentioned fatty alcohols. Preferred fatty alcohols are cetyl, stearyl, behenyl alcohols and mixtures thereof. Most preferred are stearyl alcohol, behenyl alcohol and cetylstearyl (or cetearyl) alcohol which is a mixture, usually 1 to 1 by weight, of cetyl and stearyl alcohol available for example from Cognis under the trade name Lanette O.

Concentration of fatty alcohol and in case more than one fatty alcohol is contained the total concentration of mixture of fatty alcohols, is in the range of 0.5 to 20%, preferably 1 to 15% and more preferably 2 to 10% by weight, calculated to total composition.

Compositions of the present invention comprise at least one emulsifier selected from cationic, non-ionic and amphoteric surfactants. Anionic surfactants are principally suitable emulsifiers but not preferred because of compatibility problems raise in the presence of cationic compounds and especially in the present invention with cationic polymers. Preferred surfactants as emulsifiers are cationic and non-ionic ones and mixtures thereof. All mono alkyl quaternary ammonium surfactants and all non-ionic surfactants are suitable emulsifiers for the compositions of the present invention. Preferred are mono alkyl quaternary ammonium surfactants and ethoxylated fatty alcohols.

Concentration of any of the additional conditioners mentioned above either alone or in mixture with each other can be in the range of 0.01 to 15%, preferably 0.05 - 10%, more preferably 0.1 - 5% by weight, calculated to the total composition.

The compositions according to the present invention can also comprise further agents, such as protein hydrolyzates and polypeptides, e.g. keratin hydrolyzates, collagen hydrolyzates of the type "Nutrilan" or elastin hydrolyzates, as well as, in particular vegetable, optionally cationized protein hydrolyzates, for example "Gluadin".

Additional natural plant extracts can as well form part of the compositions of the present invention. Those are incorporated usually in an amount of about 0.01 % to about 10 %, preferably 0.05 % to 7.5 %, in particular 0.1 % to 5 % by weight, calculated as dry residue thereof to the total composition. Suitable aqueous (e.g. steam-distilled) alcoholic or hydro-alcoholic plant extracts known per se are in particular extracts from leaves, fruits, blossoms, roots, rinds or stems of aloe, pineapple, artichoke, arnica, avocado, valerian, bamboo, green tea, blue lotus flower, henbane, birch, stinging nettle, echinacea, ivy, wild angelica, gentian, ferns, pine needles, silver weed, ginseng, broom, oat, rose hip, hamamelis, hay flowers, elderberry, hop, coltsfoot, currants, chamomile, carrots, chestnuts, clover, burr root, cocoanut, cornflower, lime blossom, lily of the valley, marine algae, balm, mistletoe, passion flower, ratanhia, marigold, rosemary, horse chestnut, pink hawthorn, sage, horsetail, yarrow, primrose, nettle, thyme, walnut, wine leaves, white hawthorn, etc.

Suitable trade products are, for example, the various "Extrapone" products and "Herbasol^{R} ". Extracts and the preparation thereof are also described in "Hagers Handbuch der pharmazeutischen Praxis", 4^{th} Ed.

Compositions of the present invention can comprise one or more UV filter either for stabilization of the product colour or for protection of hair from environmental influences such as loss of elasticity, loss of hair colour (bleaching effect of sun light). The UV-absorbing substance is preferably selected from the following compounds: 4-Aminobenzoic acid and the esters and salts thereof, 2-phenyl benzimidazole-5-sulfonic acid and the alkali and amine salts thereof, 4-dimethyl aminobenzoic acid and the esters and salts thereof, cinnamic acid and the esters and salts thereof, 4-methoxycinnamic acid and the esters and salts thereof, salicylic acid and the esters and salts thereof, 2.4-dihydroxybenzophenone, 2.2'.4.4'-tetrahydroxybenzophenone, 2-hydroxy-4-methoxybenzophenone and its 5-sulfonic acid or the sodium salt thereof, 2.2'-dihydroxy-4.4'-dimethoxybenzophenone, 2-hydroxy-5-chlorobenzophenone, 2.2'-dihydroxy-4-methoxybenzophenone, 2.2'-dihydroxy-4.4'-dimethoxy-5.5'-disulfobenzo-phenone or the sodium salt thereof, 2-hydroxy-4-octyloxybenzophenone, 2-hydroxy-4-methoxy-4'-methylbenzophenone, 3-benzyl-idenecampher, 3-(4'-sulfo)-benzyl-idenebornane-2-one and the salts thereof 3-(4'-methyl benzylidene)-DL-campher, and/or polysilicone-15. The preferred concentration of the UV-absorber ranges from about 0.01 % to 2.5%, more preferably from 0.05 % to 1 % by weight, calculated to the total composition.

The compositions of the present invention can comprise hair-restructuring agents. The hair restructuring agents preferred are especially the ones disclosed in the German patent DE 197 51 550 C2. Namely they are ceramide type of compounds, fatty acids and phytosterol or their mixtures.

Preferred ceramide compound is cetyl-PG-hydroxyethylpalmitamide.

Preferred fatty acids are those with 10 to 24 carbon atoms and especially with 16 to 24 carbon atoms.

Sterols, especially the phytosterols, are as well preferred hair restructuring agents as disclosed in the above mentioned german patent. Especially preferred ones are of plant origin for example ergosterol, sitosterol, stigmasterol, fucosterol, brassicasterol, fungisterol, campesterol, zymosterol, ascosterol, cerevisterol, episterol, faecosterol, spinasterol. Among those phytosterols, the ones found in "Avocadin" which is the unsaponified fraction of the avocado oil is more preferred.

The concentration of ceramide in the compositions of the present invention can be in the range of 0.01 to 2% and especially 0.01 to 1% by weight calculated to the total weight of the composition. The fatty acids may be contained at a level of 0.01 to 2.5% and especially 0.01 to 1% by weight calculated to the total weight of the composition. Phytosterol concentration of the conditioners is less than 1% and preferably in the range of 0.01 to 0.5% by weight calculated to the total weight of the composition. It should be noted without limiting the use of those ingredients the effect of those hair restructuring ingredients is especially elevated when used in combination with organic solvents as penetration enhancers.

In a further embodiment of the present invention compositions comprise at least one direct dye for colouring hair. Suitable direct dyes are cationic, anionic, neutral dyes and mixtures thereof as available commercially from various suppliers and used mainly in semi-permanent hair coloration.

One of the suitable direct dyes are cationic dyes. Non-limiting examples are Basic Blue 6, Basic Blue 7, Basic Blue 9, Basic Blue 26, Basic Blue 41, Basic Blue 99, Basic Brown 4, Basic Brown 16, Basic Brown 17, Basic Orange 31, Natural Brown 7, Basic Green 1, Basic Red 2, Basic Red 12 Basic Red 22, Basic Red 51, Basic Red 76, Basic Violet 1, Basic Violet 2, Basic Violet 3, Basic Violet 10, Basic Violet 14. Basic Yellow 57 and Basic Yellow 87, and their salts such as chloride, methosulfate, bromide etc. and mixtures thereof. Among the cationic direct dyes preferred are Basic red 51, Basic orange 31 and Basic yellow 87 and their mixture and/or their mixture with the remaining cationic dyes listed above.

Further suitable direct dyes are anionic dyes. Suitable non-limiting examples are Acid Black 1, Acid Blue 1, Acid Blue 3, Food Blue 5, Acid Blue 7, Acid Blue 9, Acid Blue 74, Acid Orange 3, Acid Orange 6, Acid Orange 7, Acid Orange 10, Acid Red 1, Acid Red 14, Acid Red 18, Acid Red 27, Acid Red 50, Acid Red 52, Acid Red 73, Acid Red 87, Acid Red 88, Acid Red 92, Acid Red 155, Acid Red 180, Acid Violet 9, Acid Violet 43, Acid Violet 49, Acid Yellow 1, Acid Yellow 23, Acid Yellow 3, Food Yellow No. 8, D&C Brown No. 1, D&C Green No. 5, D&C Green No. 8, D&C Orange No. 4, D&C Orange No. 10, D&C Orange No. 11, D&C Red No. 21, D&C Red No. 27, D&C Red No. 33, D&C Violet 2, D&C Yellow No. 7, D&C Yellow No. 8, D&C Yellow No. 10, FD&C Red 2, FD&C Red 40, FD&C Red No. 4, FD&C Yellow No. 6, FD&C Blue 1, Food Black 1, Food Black 2, Disperse Black 9 and Disperse Violet 1 and their alkali metal salts such as sodium, potassium, and their mixtures.

Further suitable dyes for colouring hair within the meaning of the present invention are those of neutral nitro dyes. Suitable non-limiting examples are HC Blue No.2, HC Blue No.4, HC Blue No.5, HC Blue No.6, HC Blue No.7, HC Blue No.8, HC Blue No.9, HC Blue No.10, HC Blue No.11, HC Blue No.12, HC Blue No.13, HC Brown No.1, HC Brown No.2, HC Green No.1, HC Orange No,1, HC Orange No.2, HC Orange No.3, HC Orange No.5, HC Red BN, HC Red No.1, HC Red No.3, HC Red No,7, HC Red No.8, HC Red No.9, HC Red No.10, HC Red No.11, HC Red No.13, HC Red No.54, HC Red No.14, HC Violet BS, HC Violet No.1, HC Violet No.2, HC Yellow No.2, HC Yellow No.4, HC Yellow No.5, HC Yellow No.6, HC Yellow No.7, HC Yellow No.8, HC Yellow No.9, HC Yellow No.10, HC Yellow No.11, HC Yellow No.12, HC Yellow No.13, HC Yellow No.14, HC Yellow No.15, 2-Amino-6-chloro-4-nitrophenol, picramic acid, 1,2-Diamino-4-nitrobenzol, 1,4-Diamino-2-nitrobenzol, 3-Nitro-4-aminophenol, 1-Hydroxy-2-amino-3-nitrobenzol and 2-hydroxyethylpicramic acid, and their mixtures.

Plant dyestuffs may also be used as hair colorant within the meaning of the present invention for example henna (red or black), alkanna root, lacoaic acid, indigo, logwood powder, madder root and rhubarb powder, etc.

It should be noted that the above dyestuffs are also suitable for use in mixture. In other words, cationic, anionic and nitro dyes are used in mixture within the meaning of the present invention. When using direct dyes of various categories, their compatibility must be taken into account.

Among the direct dyes cationic and nitro dyes are preferred ones. Most preferred ones are cationic direct dyes.

Concentration of direct dyes in the compositions of the present invention is within the range of 0.001 to 6%, preferably 0.01 to 4% and more preferably 0.05 to 3%, and most preferably 0.1 to 2% by weight, calculated to total composition.

Furthermore compositions of the present invention may comprise colour effect pigment consisting of synthetic mica coated with metal oxide or oxides and having a particle size distribution of 1 to 750 µm at a concentration of 0.01 to 10% by weight, calculated to total composition. When synthetic mica coated with metal oxide or oxides is present the preferred usage is rinse off.

The pH of the compositions according to the invention is in the range of 2 to 8, preferably 3 to 6, more preferably 3 to 5.5. For adjusting the pH of the said compositions, following ingredients can be used: Organic acids such as citric acid, lactic acid, tartaric acid, malic acid, maleic acid, fumaric acid, levulinic acid, butyric acid and hydroxy butyric acids, valeric acid, oxalic acid, succinic acid, mandelic acid, glycolic acid, glucuronic acid, propionic acid, salicylic acid or acetic acid or inorganic acids such as hydrochloric acid, phosphoric acid, sulphuric acid, nitric acid. Concentration of the organic and/or inorganic acids or their mixtures should be chosen in a way that composition reaches the desired pH value as given above. Typically concentration for acids can be 0.01 - 3% by weight, preferably 0.05 - 2% by weight, more preferably 0.05 - 1.5% by weight calculated to the total composition. The pH of the composition can also be adjusted to the required pH by using alkaline solution such as sodium hydroxide, ammonium hydroxide, potassium hydroxide or their salts with those acids mentioned above in the case that at the selected acid concentration pH of the composition is lower than that of the aimed value.

Furthermore compositions of the present invention can comprise all substances customarily found in such preparations. Examples of such substances are complexing agents, preservatives, fragrances, etc.

Compositions of the present invention are suitable for use both as rinse off and leave in especially after cleansing hair. In case of leave in usage, use without previous cleansing is not excluded.

Accordingly, process for conditioning hair wherein hair is cleansed with a suitable cleansing composition as usual and subsequently a composition comprising at least one cationic cellulose polymer and at least one cationic or cationizable silicone compound is applied onto hair and after processing of 30 sec to 30 min at a temperature of 20 to 45°C rinsed off from hair.

Further, process for conditioning hair according to the present invention is application of a conditioning composition comprising at least one cationic cellulose polymer and at least one cationic or cationizable silicone compound onto cleansed or wetted or dry hair and is not rinsed of from hair.

In a trial it has further been found out that hair conditioning properties of the compositions of the present invention is particularly elevated when hair cleansed with a cleansing composition comprising as well cationic cellulose polymer and cationic or cationizable silicone compound. Accordingly, further subject of the present invention is kit for cleansing and conditioning hair which comprises a cleansing composition comprising at least one cationic cellulose polymer and at least one cationic or cationizable silicone compound and a composition comprising at least one cationic silicone polymer and at least one cationic or cationizable silicone compound.

Following examples are to illustrate the invention but not to limit.

### Example 1

***: Used as raw material DC 8500, the number in the formula refers to active matter.**

| | % by weight |
|---|---|
| Polyquaternium-67 | 0.50 |
| Bis (C13-15 alkoxy) PG-amodimethicone* | 0.75 |
| Hydroxyethylcellulose | 1.0 |
| Citric acid | q.s. to pH 4.5 |
| Fragrance, preservative | q.s. |
| Water | q.s. to 100 |

The above composition can be used as a rinse off composition and also as a leave-in composition. Under both usage condition it was observed that the above composition improves combability, gives hair shine, especially in rinse of usage and give hair volume and body (body is very much enhanced especially in leave-in usage) and makes hair manageable. It was also observed that hair is possible to style easily and style holds reasonably long period of time.

### Example 2

***: Used as raw material DC 8500, the number in the formula refers to active matter.**

| | % by weight |
|---|---|
| Stearyl alcohol | 7.0 |
| Cetrimonium chloride | 1.0 |
| Polyquaternium-10 | 0.75 |
| Bis (C13-15 alkoxy) PG-amodimethicone* | 0.75 |
| Citric acid | q.s. to pH 4.5 |
| Fragrance, preservative | q.s. |
| Water | q.s. to 100 |

The above composition was prepared by emulsifying stearyl alcohol, cetrimonium chloride, and bis (C13-15 alkoxy) PG-amodimethicone*at approximately 70°C in part of water and subsequently after cooling down to around 40°C, solution of polyquaternium 10 in water, and fragrance were added. Finally pH was adjusted.

For comparative purpose the above composition was prepared by replacing the Bis (C13-15 alkoxy) PG-amodimethicone with the same amount of dimethicone.

The above composition was tested in a half side test with 10 consumers having shoulder length damaged hair against the comparative composition. Before application of the above composition, hair was washed with a commercially available shampoo. Afterwards to the half side 8 g of the above composition of Example 1 and comparative composition were applied and processed for 5 min at ambient temperature. At the end of the processing time the hair was rinsed off wit tap water at approximately 40°C. The hair was towel dried and dried with a hair drier. The hair was evaluated in wet (before drying) and dry states by hair dressers (at least 2) and customers preference was asked. The following results were obtained.

**Table I: Results of half side comparative test of Example 2**

| | Example 2 | Comparative composition | No difference |
|---|---|---|---|
| Wet hair | | | |
| Combability | 6 | 3 | 1 |
| Smoothness | 7 | 3 | 0 |
| Roughness | 0 | 1 | 9 |
| Dry hair | | | |
| Combability | 7 | 2 | 1 |
| Smoothness | 7 | 2 | 1 |
| Roughness | 1 | 2 | 7 |
| Shine | 7 | 2 | 1 |
| Volume | 8 | 2 | 0 |
| Body | 5 | 3 | 2 |
| Elasticity | 7 | 2 | 1 |
| Preference | 6 | 2 | 2 |

From the above results it is clear that conditioning properties of the inventive composition is much better than the comparative composition.

### Example 3

| | % by weight |
|---|---|
| Cetearyl alcohol | 6.0 |
| Cteareth-20 | 2.0 |
| Polypuaternium-70* | 0.4 |
| Polyquaternium-24 | 0.8 |
| Amodimethicone** | 1.0 |
| Citric acid/Sodium hydroxide | q.s. to pH 4.0 |
| Fragrance | 0.2 |
| Water | to 100 |

| | |
|---|---|
| *: used as Lustreplex, the concentration in the composition refers to active matter. ** DC 949, the number represents amodimethicone active matter | |

The above composition was prepared in the same way as the example 1. The above composition is suitable for leave-in and rinse of usages.

Upon use on fine hair it was found out that hair is excellently combable and feels smooth, has improved shine, elasticity and volume and body.

### Example 4

| | % by weight |
|---|---|
| Cetearyl alcohol | 8.0 |
| Cteareth-20 | 3.0 |
| Stearamidopropyl trimonuim chloride | 1.0 |
| Polyquaternium-67 | 0.4 |
| Quaternium-80 | 0.3 |
| Benzophenone-4 | 0.3 |
| Citric acid/Sodium hydroxide | q.s. to pH 3.5 |
| Fragrance | 0.2 |
| Water | to 100 |

### Example 5

| | % by weight |
|---|---|
| Behenyl alcohol | 9.0 |
| Behentrimonuim chloride | 2.0 |
| Bis (C13-15 alkoxy) PG-amodimethicone* | 0.5 |
| Polyquaternium-67 | 0.5 |
| Citric acid/Sodium hydroxide | q.s. to pH 4.0 |
| Fragrance | 0.2 |
| Water | to 100 |

### Example 6

| | % by weight |
|---|---|
| Behenyl alcohol | 7.0 |
| Behentrimonuim chloride | 1.0 |
| Ceteareth-20 | 1.0 |
| Polyquaternium-67 | 0.4 |
| Bis (C13-15 alkoxy) PG-amodimethicone* | 0.5 |
| Ethyl hexyl methoxycinnamate | 0.2 |
| Citric acid/Sodium hydroxide | q.s. to pH 3.5 |
| Fragrance | 0.2 |
| Water | to 100 |

### Example 7

| | % by weight |
|---|---|
| Behenyl alcohol | 8.0 |
| Behentrimonuim chloride | 1.0 |
| Bis (C13-15 alkoxy) PG-amodimethicone | 0.5 |
| Polyquaternium-67 | 0.4 |
| Benzophenone-4 | 0.3 |
| Citric acid/Sodium hydroxide | q.s. to pH 3.5 |
| Fragrance | 0.2 |
| Water | to 100 |

### Example 8

| | % by weight |
|---|---|
| Cetearyl alcohol | 10.0 |
| Behentrimonuim chloride | 1.0 |
| Ceteareth-20 | 1.0 |
| Polyquaternium-67 | 0.4 |
| Bis (C13-15 alkoxy) PG-amodimethicone* | 0.5 |
| Dimethicone** | 1.0 |
| Benzophenone-3 | 0.3 |
| Benzophenone-4 | 0.3 |
| Citric acid/Sodium hydroxide | q.s. to pH 3.5 |
| Fragrance | 0.2 |
| Water | to 100 |

| | |
|---|---|
| **: DC 200 with a viscosity of 60,000 mPa.s. | |

### Example 9

| | % by weight |
|---|---|
| Cetearyl alcohol | 9.0 |
| Ceteareth-20 | 3.0 |
| Polyquaternium-67 | 0.4 |
| Amodimethicone** | 0,5 |
| Benzophenone-3 | 0.3 |
| Citric acid/Sodium hydroxide | q.s. to pH 3.5 |
| Fragrance | 0.2 |
| Water | to 100 |

| | |
|---|---|
| **: DC 949, the concentration refers to active amodimethicone concentration | |

### Example 10

| | % by weight |
|---|---|
| Cetearyl alcohol | 9.0 |
| Behentrimonium chloride | 1.5 |
| Ceteareth-20 | 3.0 |
| Polyquaternium-67 | 0.4 |
| Bis (C13-15 alkoxy) PG-amodimethicone* | 0.5 |
| Benzophenone-3 | 0.3 |
| Basic red 51 | 0.1 |
| Citric acid/Sodium hydroxide | q.s. to pH 3.5 |
| Fragrance | 0.2 |
| Water | to 100 |

### Example 11

| | % by weight |
|---|---|
| Cetearyl alcohol | 9.0 |
| Behentrimonium chloride | 1.5 |
| Ceteareth-20 | 3.0 |
| Polyquaternium-67 | 0.4 |
| Bis (C13-15 alkoxy) PG-amodimethicone* | 0.5 |
| Benzophenone-3 | 0.3 |
| Basic red 51 | 0.1 |
| Basic red 76 | 0.1 |
| Citric acid/Sodium hydroxide | q.s. to pH 3.5 |
| Fragrance | 0.2 |
| Water | to 100 |

### Example 12

| | % by weight |
|---|---|
| Cetearyl alcohol | 9.0 |
| Behentrimonium chloride | 1.5 |
| Ceteareth-20 | 3.0 |
| Polyquaternium-70* | 0.4 |
| Bis (C13-15 alkoxy) PG-amodimethicone | 0.5 |
| Benzophenone-3 | 0.3 |
| Basic orange 31 | 0.04 |
| Basic yellow 87 | 0.1 |
| Citric acid/Sodium hydroxide | q.s. to pH 3.5 |
| Fragrance | 0.2 |
| Water | to 100 |

## Claims

1. An aqueous composition for conditioning keratin fibres especially human hair **characterised in that** it comprises at least one cationic cellulose polymer and at least one cationic or cationizable silicone compound.

2. Composition according to claim 1 at least one cationic cellulosic polymer is chosen from Polyqauternium 4, Polyqauternium 10, Polyqauternium 24, Polyqauternium 67 and Polyqauternium 72.

3. Composition according to any of the preceding claims **characterised in that** at least one cationizable or cationic silicone compound is chosen from amodimethicone, quaternium-80, quaternised graft polymers of organopolysiloxane and polyethyloxazoline, and bis (C13-15 alkoxy) PG-amodimethicone.

4. Composition according to any of the preceding claims **characterised in that** at least one cationizable or cationic silicone compound is bis (C13-15 alkoxy) PG-amodimethicone.

5. Composition according to any of the preceding claims **characterised in that** it is an emulsion or a thickened gel.

6. Composition according to any of the preceding claims it comprises at least one fatty alcohol and at least one emulsifier selected from cationic, non-ionic and amphoteric surfactants.

7. Composition according to claim 6 **characterised in that** at least one emulsifier is a cationic surfactant of following general formula 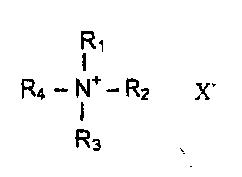 where R₁ is a saturated or unsaturated, branched or non-branched alkyl chain with 8-22 C atoms or
R₅CO NH (CH₂)ₙ
where R₅ is saturated or unsaturated, branched or non-branched alkyl chain with 7-21 C atoms and n has typical value of 0 - 4 or
R₆CO O (CH₂)ₙ
where R₆ is saturated or unsaturated, branched or non-branched alkyl chain with 7-21 C atoms and n has typical value of 0 - 4, and
and R₂, R₃ and R₄ are independent from each other H or lower alkyl chain with 1 to 4 carbon atoms or ethoxy or propoxy group with number of ethoxy or propoxy groups varying in the range of 0 to 4, and X is chloride, bromide or methosulfate.

8. Composition according to claims 6 and 7 **characterised in that** at least one emulsifying surfactant is non-ionic surfactant and selected from alkyl polyglucosides of the general formula
R₇ - O - (R₈O)_{N} O - Zₓ
wherein R₇ is an alkyl group with 8 to 18 carbon atoms, R₈ is an ethylene or propylene group, Z is a saccharide group with 5 to 6 carbon atoms, n is a number from 0 to 10 and x is a number between 1 and 5, long chain fatty acid mono or dialkanolmaides, sorbitan esters and C₁₀-C₂₂-fatty alcohol ethoxylates.

9. Composition according to any of the preceding claims **characterised in that** it comprises at least one additional conditioning agent selected from cationic surfactants, oils either synthetic or natural, cationic polymers.

10. Composition according to any of the preceding claims **characterised in that** it comprises at least one UV filter.

11. Composition according to any of the preceding claims **characterised in that** it comprises at least one direct dye.

12. Use of compositions according to claims 1 to 11 for conditioning hair.

13. Process for conditioning hair wherein hair is cleansed with a suitable cleansing composition as usual and subsequently a composition according to claims 1 to 13 is applied onto hair and after processing of 30 sec to 30 min at a temperature of 20 to 45°C rinsed off from hair.

14. Process for conditioning hair wherein a composition according to claims 1 to 13 is applied onto cleansed or wetted or dry hair and without rinsing off hair is dried.

15. Kit for cleansing and conditioning hair **characterised in that** it comprises a cleansing composition and a conditioning composition according to ayn of the claims 1 to 11.
